# EUROPEAN PATENT APPLICATION

(11) **EP 4 113 368 A1**
(43) Date of publication of application: **04.01.2023**
(21) Application number: 21717943.1
(22) Date of filing: 09.02.2021
(51) Int. Cl.: G06K 9/00, C12M 1/00

(54) **SYSTEM FOR CONTROLLING THE DEVELOPMENT OF A CULTURE IN A SOLID MEDIUM IN A ROBOTISED INCUBATOR**

(30) Priority: 28.02.2020 ES 202030170
(71) Applicant: Sener Aeroespacial S.A.U., 48930 Getxo - Vizcaya (ES)
(72) Inventor: JOAN, Ariño Bringué, 48930 GETXO - VIZCAYA (ES)
(74) Representative: Clarke Modet & Co.
(86) International application number: PCT/ES2021/070089
(87) International publication number: WO 2021/170889

(57) **Abstract**

The present invention relates to a system for controlling the development of a culture in solid medium in a robotic incubator (100), which comprises an imaging system (1) configured to take images without moving a Petri dish (5) from the location wherein the culture is carried out, a system for analysing the images obtained by the imaging system, wherein based on the information provided by the system for analysing images from the imaging system, which analyses the images provided by the imaging system by means of a growth detection algorithm, the development of the culture in an incubator (100) without moving the Petri dish (5) from the location wherein the culture is carried out is disclosed.

## Description

### Field of the invention

The present invention relates to a system for controlling the development of the culture in solid medium in a robotic incubator that enables the monitoring and control of said development in solid medium without the need to move the Petri dish from the location wherein the culture is carried out. The system for controlling the development of the culture in solid medium in an incubator object of the invention is applicable in the field of laboratories wherein biological cultures of microorganisms are developed and studied.

### Background of the invention

There are clinical studies wherein it is necessary to carry out a microbiological or cell culture and to carry out said culture, automatic incubators are used, which are devices able to maintain and grow microbiological or cell cultures maintaining conditions of temperature, humidity and O2 or CO2 content present.

Many of the automatic incubators for application in microbiology have an imaging system, which enables each one of the dishes to be periodically analysed in order to determine how the processing thereof continues according to whether there is growth or not, assessing different parameters, among others, the image of the dish and the incubation time.

There are two ways to develop a culture in a test, it can be developed in liquid medium and in solid medium. Controlling the growth or development of a test in liquid medium is relatively simple, since the liquids are more or less homogeneous and the control systems in liquid mediums are based on controlling features of the homogeneous liquid, such as, for example, the fluorescence, which is different if bacteria are present or not in the liquid. However, in cultures developed in solid medium, growth control is completely different since it is necessary to search for non-homogeneities in the solid medium, since these non-homogeneities are those which indicate areas with bacterial growth.

In tests in liquid medium, detection based on optical systems is widespread as it is easier to identify growth or development, since it is based on searching for homogeneities of the liquid medium.

In the state of the art, the analysis of the test dishes in solid medium is done periodically, by means of an imaging system, based on an incubation time pattern, but for imaging, it is necessary to extract the Petri dish from inside the incubator and, at that point, the Petri dish loses the optimal test conditions thereof.

Document WO2017185012A1 is known in the state of the art, which relates to an automated system for an incubator of microorganisms that enables the different tests carried out on the cultures to be controlled in tests carried out on wells, which are a liquid medium, said system consists of a housing for multiple well dishes, containing samples, manipulated with a robotic arm, and an optical subsystem to monitor the growth of cultures. This last element includes an illumination source (for example, halogen, LED or laser...) and at least one detector (for example CCD, CMOS, photodiode sensor technology...).

However, this system, when applied for controlling growth in a liquid medium, bases growth control on the search for homogeneous features of the liquid, such as fluorescence, which are different if there are bacteria in the liquid medium or not.

In the state of the art, the WASP LAB - Smart Incubator is known, which has an imaging system that takes an image of the dish by moving the dish to a position in which the imaging system is located and that bases the search for growth of the culture on the dish on the time pattern, so that it moves the dishes from time to time to the imaging position, and based on these images, the growth or not of the culture in the dish is assessed.

This system has a number of drawbacks:
- The dishes are analysed by the imaging system based on the time pattern, generally every 4 hours. The growth of bacteria or other organisms can have highly variable time patterns, from a few minutes to several days;
- The use of the time pattern is not very representative;
- The imaging systems known in the state of the art require removing the Petri dish from the incubator with the consequent loss of time and limitation to the maximum capacity of the incubator;
- The incubator cannot contain more dishes than the imaging system is capable of processing every 4 hours;
- For a usual number of dishes of the order of 800-1000, the period of revision of the dishes cannot go below 3-4 hours;
- During the time that the dishes are out of the incubator to obtain the images, the dishes are not in the expected incubation conditions, therefore the test does not develop in optimal conditions;
- The continuous movement of dishes entering and leaving the incubator causes disturbances in the incubation conditions.

In the same way, this system sends the image to a monitor wherein a technician assesses whether or not there is development of the culture under study.

In the state of the art, document WO2014008222A1 is known, which discloses a device configured for selecting cells or groups of cells within a heterogeneous sample that requires a previous chemical treatment on the sample before making the detection, while with the device object of the invention the sample is not altered with any treatment.

### Description of the invention

The object of the invention is a system for controlling the development of the culture in solid medium in a robotic incubator that comprises an imaging system configured to take images without moving the Petri dish from the location wherein the culture is carried out, and a system for analysing the images obtained by the imaging system, wherein based on the information provided by the system for analysing images from the imaging system, which analyses the images provided by the imaging system by means of a growth detection algorithm, the development of the culture in an incubator without moving the Petri dish from the location wherein the culture is carried out is disclosed.

Several options are envisaged for the growth detection algorithm of the system for controlling the development of the culture in solid medium in a robotic incubator object of the invention:
- one is based on the identification of differences of the images with respect to an initial image, taken at time zero where there is no growth,
- another option is based on artificial intelligence wherein this growth detection algorithm is trained with Deep Learning techniques to be able to classify the images according to the level of growth they have,
- another option is mixed techniques wherein the artificial intelligence growth detection algorithm also uses the image at time zero.

In the system for controlling the development of the culture in solid medium in a robotic incubator object of the invention, the imaging system comprises a camera configured to take images directly on the Petri dishes.

In another embodiment, the imaging system of the system for controlling the development of the culture in solid medium in a robotic incubator object of the invention, comprises a camera and at least one mirror configured to provide the camera with focus on the Petri dish for imaging.

In the system for controlling the development of the culture in solid medium in a robotic incubator object of the invention, the imaging system comprises an illumination system configured to illuminate the Petri dish inside the incubator and facilitate imaging by the aforementioned imaging system.

The illumination system comprises at least one of the following elements: light sources inside the incubator, a spotlight outside the incubator focussed on the Petri dish, and an optical guide system that guides the light up to the Petri dish.

Images can be taken directly from the face of the Petri dish wherein growth has taken place or from the face of the subsequent Petri dish. In this case the illumination system projects the growth to the rear portion of the Petri dish from which the image is taken.

### Description of the drawings

To complement the description that is going to be made below and in order to help a better understanding of the features of the invention, this description is accompanied by a set of drawings based on which the innovations and advantages of the object of the invention will be more easily understood.

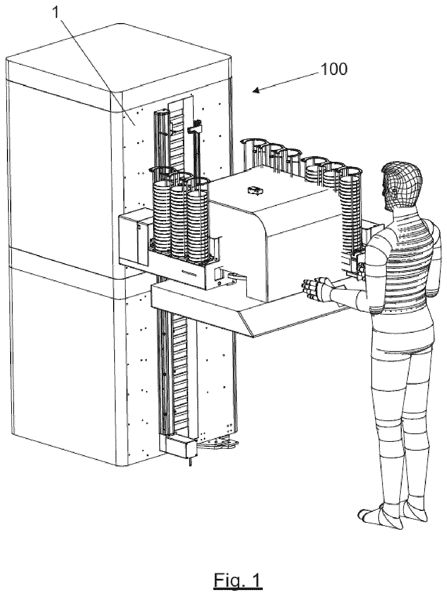

Figure 1 shows a perspective view of a robotic incubator for culture in solid medium with the system for controlling the development of the culture object of the invention.

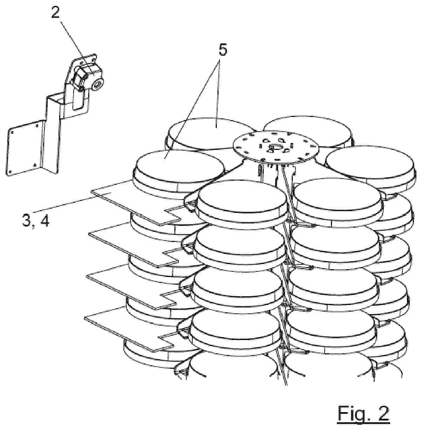

Figure 2 shows a perspective view of the imaging system of the system for controlling the development of the culture object of the invention.

### Detailed description of the invention

Before defining in detail the system for controlling the development of the culture in solid medium in a robotic incubator (100) object of the invention, it is necessary to emphasise that the culture in solid medium is developed on Petri dishes (5), which are appropriate for this type of culture.

The system for controlling the development of the culture in solid medium in a robotic incubator (100) object of the invention comprises:
- an imaging system (1) configured to take images of the Petri dishes (5) inside the incubator (100);
- a system for analysing the images taken by the imaging system (1), configured to determine the existence or not of growth of a culture in a Petri dish (5).

The imaging system (1) is configured to take images of the Petri dishes (5), that is, the sample in a solid medium, periodically, but, unlike the imaging systems for control of the development of a culture in solid medium known in the state of the art, these images are taken without the need to move the Petri dish (5) with the culture in solid medium from the location wherein the culture is carried out.

To know the dishes that are developing correctly according to the test being carried out, the system for analysing images of the system for controlling the development of a culture in an incubator (100) object of the invention uses a growth detection algorithm that assesses the possible growth or not of a culture based on the aforementioned photograph.

For the growth detection algorithm, the system for controlling the development of the culture in solid medium in a robotic incubator (100) object of the invention envisages several options:
- one is based on the identification of differences of the images with respect to an initial image, taken at time zero where there is no growth;
- another option is based on artificial intelligence wherein the growth detection algorithm is trained with Deep Learning techniques to be able to classify the images according to the level of growth they have;
- another option is mixed techniques wherein the artificial intelligence growth detection algorithm also uses the image at time zero.

The trained growth detection algorithm works based on a library of images classified in interest groups, carried out by a specialist, which is used to train the growth detection algorithm by teaching it the classification based on the image, so that, once the classified image library has been taught, the growth detection algorithm has the ability to classify new images automatically. It is necessary to review the classification carried out by the growth detection algorithm, because if during nominal operation any image not classified correctly is detected, this erroneously classified image is used *a posteriori* to improve the training of the growth detection algorithm.

In the system for controlling the development of the culture in solid medium in a robotic incubator (100) object of the invention, the most basic classification is carried out in two groups (growth and no growth), although it can be extended to several groups classified by different growth levels.

For the imaging of the imaging system (1), said imaging system (1) comprises a camera (2) that can take the images in two different ways:
- directly on the Petri dishes (5), (100), or
- by using mirrors (3) to focus on the Petri dish (5).

Similarly, there are two options for taking images, images of the Petri dish (5) can be taken, either from the top or from the bottom portion thereof.

The images taken by the imaging system (1) are taken in a continuous cycle, one Petri dish (5) after another, taking into account two limitations:
- the ability to move of the camera (2), and
- biological criteria of culture growth, since it does not make any sense to take images every minute since the cultures do not develop as fast.

The imaging system also has an illumination system (4) that, together with the mirrors (3) or the orientation of the camera (2), facilitates imaging by the aforementioned imaging system. As the images are taken, the illumination system (4) can illuminate the Petri dish (5) from above or illuminate the Petri dish (5) from below.

The Petri dishes (5), inside the incubator, are separated from each other, in height, so that the gap between Petri dish (5) and Petri dish (5) is used to illuminate them and obtain the images either by direct vision or by means of a mirror (3).

There are different options of the illumination system since said system may comprise light sources inside the incubator (100), a spotlight outside pointing to the Petri dish (5) or can be an optical guide system (optical fibre) that guides the light up to the Petri dish (5).

Thanks to the images from the imaging system and to the system for analysing the images obtained by the imaging system, the system for controlling the development of the culture in an incubator (100) makes it possible to exclusively act on those Petri dishes (5) wherein the culture is being developed according to the requirements of a test or, on the contrary, discard those Petri dishes (5) that do not comply with the provisions of the test, without affecting the development conditions of the other Petri dishes (5).

## Claims

1. A system for controlling the development of a culture in solid medium in a robotic incubator (100), **characterised in that** it comprises:
- an imaging system (1), comprising a mobile camera (2) configured to take images without moving a Petri dish (5) from the location wherein the culture is carried out at least one way to choose between:
- directly on the Petri dishes (5), (100), or
- by using at least one mirror (3) to focus on the Petri dish (5),
- a system for analysing the images obtained by the imaging system comprising a processor configured to process images,
wherein the processor of the system for analysing the images processes the images from the imaging system and by means of a growth detection algorithm provides information on the development of the culture in an incubator (100) without moving the Petri dish (5) from the location wherein the culture is carried out.

2. The system for controlling the development of the culture in solid medium in a robotic incubator (100) according to claim 1, **characterised in that** the growth detection algorithm is an option to choose between:
- identification of differences of the images with respect to an initial image, taken at time zero where there is no growth;
- an artificial intelligence growth detection algorithm trained with Deep Learning techniques is able to classify the images according to the level of growth they have;
- a mixed technique wherein the artificial intelligence growth detection algorithm also uses the image at time zero.

3. The system for controlling the development of the culture in solid medium in a robotic incubator (100) according to any of claims 1 to 2 **characterised in that** the imaging system (1) comprises an illumination system (4) configured to illuminate the Petri dish (5) inside the incubator (100) and facilitate imaging by the aforementioned imaging system (1).

4. The system for controlling the development of the culture in solid medium in a robotic incubator (100) according to claim 3 **characterised in that** the illumination system (4) comprises at least one of the following elements:
- light sources inside the incubator (100),
- a spotlight outside the incubator (100) focussed on the Petri dish (5), and
- an optical fibre system that guides the light up to the Petri dish (5).
